# EUROPÄISCHE PATENTANMELDUNG

(11) **EP 3 454 069 A1**
(43) Veröffentlichungstag der Anmeldung: **13.03.2019**
(21) Anmeldenummer: 17189602.0
(22) Anmeldetag: 06.09.2017
(51) Int. Cl.: G01R 33/48, G01R 33/421, A61B 6/00

(54) **BILDGEBUNGSSYSTEM MIT EINEM MRT-SYSTEM UND EINER RÖNTGENRÖHRE**

(71) Anmelder: Siemens Healthcare GmbH, 91052 Erlangen (DE)
(72) Erfinder: Fritzler, Anja, 91341 Röttenbach (DE); Niederlöhner, Daniel, 91054 Erlangen (DE); Weber, Thomas, 91353 Hausen (DE)

(57) **Zusammenfassung**

Die Erfindung betrifft ein Bildgebungssystem, das ein MRT-Gerät (1) für eine MR-Bildgebung und eine Röntgenröhre (2) für eine Röntgenbildgebung umfasst, wobei die Röntgenröhre (2) im Wirkungsbereich eines vom MRT-Gerät (1) erzeugten MRT-Magnetfeldes angeordnet ist und durch eine Abschirmeinrichtung (8) sowohl die Röntgenröhre (2) während der Röntgenbildgebung vor dem MRT-Magnetfeld als auch das MRT-Gerät (1) während der MR-Bildgebung vor Einflüssen der Röntgenröhre (2) zumindest teilweise abgeschirmt ist. Ein derartiges Bildgebungssystemermöglicht sowohl eine verbesserte MR-Bildgebung als auch eine verbesserte Röntgenbildgebung.

## Beschreibung

Die Erfindung betrifft ein Bildgebungssystem.

Der Betrieb eines Röntgenstrahlers in einem Magnetfeld, z.B. dem Streufeld eines Magnetresonanztomografie(MRT)-Geräts, ist nicht ohne weiteres möglich. Zum einen wird der Elektronenstrahl, welcher die Röntgenstrahlung die beim Auftreffen auf eine Drehanode erzeugt wird, im Magnetfeld des MRT-Geräts (MRT-Magnetfeld) abgelenkt. Diese Ablenkung des Elektronenstrahls kann dazu führen, dass der Elektronenstrahl die Drehanode möglicherweise nicht mehr auf der Brennbahn trifft. Zum anderen werden durch das MRT-Magnetfeld beim Betrieb der Drehanode Wirbelströme verursacht, die die Drehanode einerseits unnötig aufheizen und andererseits dem Drehanoden-Antrieb entgegenwirken. Darüber hinaus beeinflusst ein Röntgenstrahler, welcher ferromagnetische Materialien enthält, massiv die Homogenität des MRT-Magnetfelds, wodurch die Bildqualität entsprechend negativ beeinflusst wird.

Aus der US 6,810,110 B2 ist ein Bildgebungssystem bekannt, das ein MRT-Gerät (Magnetresonanztomografie-Gerät) und einen Röntgenstrahler umfasst. Der Röntgenstrahler ist im Wirkungsbereich eines vom MRT-Gerät erzeugten Magnetfeldes angeordnet und weist eine Strahlerbschirmung gegen das vom MRT-Gerät während der MR-Bildgebung erzeugte Magnetfeld auf. Bei der Abschirmung des Röntgenstrahlers handelt es sich um eine aktive Abschirmung mittels einer Spule. Der Einfluss ferromagnetischer Materialien auf die Homogenität des vom MRT-Gerät erzeugten Magnetfeldes wird mit dieser Abschirmung allerdings nicht gelöst. Somit verbleibt bei dem Bildgebungssystem gemäß der US 6,810,110 B2 als einzige Möglichkeit, sämtliche ferromagnetischen Materialien aus dem Röntgenstrahler zu eliminieren und z.B. durch Edelstahl zu ersetzen, das kaum ferromagnetisch ist.

Aus der US 6,352,363 B1 ist ein magnetisches Chirurgiesystem mit einem magnetisch navigierbaren medizinischen Gerät bekannt. Das medizinische Gerät ist in einem Körper eines Patienten magnetisch navigierbar. Zu Steuerung des medizinischen Geräts innerhalb des Körpers erzeugt das Chirurgiesystem hierzu zeitlich veränderbare Magnetfelder. Weiterhin weist das bekannte Chirurgiesystem ein Bildgebungsgerät mit wenigstens einer Röntgenröhre auf. Die Röntgenröhre ist durch ein Gussgehäuse aus einem Material auf Eisenbasis gegen die Magnetfelder abgeschirmt, die während der Navigation des medizinischen Geräts vom Chirurgiesystem erzeugt werden. Bei der Abschirmung handelt es sich somit eine passive Abschirmung, die gleichzeitig das Strahlergehäuse für die Röntgenröhre bildet. Das Gussgehäuse (Strahlergehäuse) besteht aus einem magnetisch hoch-permeablen bzw. ferromagnetischen Material. Die in der US 6,352,363 B1 vorgeschlagene magnetische Abschirmung des Bildgebungsgeräts ist für einen Einsatz in Verbindung mit einem MRT-Gerät (Magnetresonanztomografie-Gerät) nicht geeignet, da - auch bei komplett ausgeschalteter Röntgenröhre - durch die Abschirmung das von einem MRT-Gerät benötigte möglichst homogene Magnetfeld stark gestört wird. Dies resultiert allein aus der Tatsache, dass sich stark magnetisierbare Materialien im Wirkungsbereich des MRT-Geräts befinden.

Aufgabe der vorliegenden Erfindung ist es, ein Bildgebungssystem der eingangs genannten Art zu schaffen, das sowohl eine verbesserte MR-Bildgebung als auch eine verbesserte Röntgenbildgebung ermöglicht.

Die Aufgabe wird erfindungsgemäß durch ein Bildgebungssystem nach Anspruch 1 gelöst. Vorteilhafte Ausgestaltungen des erfindungsgemäßen Bildgebungssystems sind jeweils Gegenstand von weiteren Ansprüchen.

Das Bildgebungssystem gemäß Anspruch 1 umfasst ein MRT-Gerät für eine MR-Bildgebung und eine Röntgenröhre für eine Röntgenbildgebung, wobei die Röntgenröhre im Wirkungsbereich eines vom MRT-Gerät erzeugten MRT-Magnetfelds angeordnet ist. Erfindungsgemäß ist durch eine Abschirmeinrichtung sowohl die Röntgenröhre während der Röntgenbildgebung vor dem MRT-Magnetfeld als auch das MRT-Gerät während der MR-Bildgebung vor Einflüssen der Röntgenröhre zumindest teilweise abgeschirmt.

Dadurch, dass durch eine Abschirmeinrichtung sowohl die Röntgenröhre während der Röntgenbildgebung vor dem MRT-Magnetfeld als auch das MRT-Gerät während der MR-Bildgebung vor Einflüssen der Röntgenröhre zumindest teilweise abgeschirmt ist, wird das Magnetfeld im Inneren der Röntgenröhre bzw. im Inneren des Röntgenstrahlers entsprechend unterdrückt. Gleichzeitig wird das Magnetfeld des MRT-Geräts nicht durch ferromagnetische Materialien, die in der Röntgenröhre bzw. im Röntgenstrahler angeordnet sind, gestört.

Bei dem Bildgebungssystem nach Anspruch 1 können als Röntgenröhren sowohl Röntgenröhren mit Stehanoden als auch Röntgenröhren mit Drehanoden Verwendung finden.

Bei dem erfindungsgemäßen Bildgebungssystem ist sichergestellt, dass das Magnetfeld im Inneren der Röntgenröhre bzw. im Inneren des Röntgenstrahlers unterdrückt bzw. abgeschirmt wird, wobei gleichzeitig das Magnetfeld des MRT-Geräts nicht durch ferromagnetische Materialien gestört wird.

Im Rahmen der Erfindung kann die Röntgenröhre im MRT-Gerät (Anspruch 2) oder außen am MRT-Gerät angeordnet (Anspruch 3) sein.

Bei einer besonders vorteilhaften Ausgestaltung des Bildgebungssystems umfasst die Abschirmeinrichtung wenigstens eine Spule, die die Röntgenröhre zumindest teilweise umschließt (Anspruch 4). Man erhält dadurch auf einfache Weise eine besonders wirkungsvolle aktive Abschirmung.

Ist die Röntgenröhre in einem Strahlergehäuse angeordnet (Anspruch 5), dann sind wenigstens eine Spule der Abschirmeinrichtung innerhalb des Strahlergehäuses und/oder wenigstens eine Spule Abschirmeinrichtung außerhalb des Strahlergehäuses angeordnet.

Befindet sich die Abschirmeinrichtung innerhalb des Strahlergehäuses, dann können Elemente der Abschirmeinrichtung z.B. zumindest teilweise direkt auf der Röntgenröhre angeordnet sein und bei einer Ausgestaltung als Spule beispielsweise die Röntgenröhre teilweise oder vollständig umschließen (Anspruch 4). Alternativ oder zusätzlich ist es auch möglich, Elemente der Abschirmeinrichtung (z.B. Spulen) zumindest teilweise auf der Innenseite des Strahlergehäuses anzuordnen.

Umfasst die außerhalb des Strahlergehäuses angeordnete Abschirmeinrichtung wenigstens eine Spule, dann ist es vorteilhaft, wenn eine oder mehrere dieser Spulen das Strahlergehäuse teilweise oder vollständig umschließt (Anspruch 6). Man erhält dadurch ebenfalls auf einfache Weise eine besonders wirkungsvolle aktive Abschirmung.

Für bestimmte Anwendungsfälle ist es vorteilhaft, wenn die Abschirmeinrichtung eine passive Abschirmung und eine aktive Abschirmung umfasst (Anspruch 7).

Die besonders vorteilhaften Ausgestaltungen der Bildgebungssysteme umfassen somit wenigstens eine Abschirmeinrichtung mit zumindest einer aktiven Abschirmung, wobei die aktive Abschirmung jeweils mindestens eine stromdurchflossene Spule aufweist (Ansprüche 4, 6 und 7).

Die aktive Abschirmung während des Betriebs des Magnetresonanztomografie(MRT)-Geräts wird dazu benutzt, die Störungen, die durch die im Röntgenstrahler verbliebenen ferromagnetischen Materialien verursacht werden, aktiv auszugleichen. Der Röntgenstrahler kann weiterhin mittels der aktiven Abschirmung während des Betriebs vor dem Einfluss des Magnetfelds geschützt werden. Vorteil dieser Art der Abschirmung ist, dass kein hochpermeables Material als Schirmmaterial benutzt werden muss und somit der Einfluss dieser Materialien auf das Magnetfeld des MRT-Geräts entfällt.

Eine solche aktive Abschirmung ist z.B. mithilfe eines Gehäuses in welchem eine oder mehrere stromdurchflossene Spulen verbaut sind erfolgen. Das Gehäuse könnte z.B., ähnlich der aus einem MRT-Gerät bekannten Gradientenspulen, mittels Vergusstechnik gefertigt werden. Vorteil dieser Art der Abschirmung ist, dass kein hochpermeables Material als Schirmmaterial benutzt werden muss und somit der Einfluss dieser Materialien auf das Magnetfeld des MRT-Geräts entfällt. Solange der Röntgenstrahler außer Betrieb ist bzw. keine dynamischen elektromagnetischen Felder (EM-Felder) erzeugt, kann die Beeinflussung des MRT-Magnetfelds dadurch minimiert werden. Eine Beeinflussung des MRT-Magnetfelds findet somit nur zu dem Zeitpunkt statt, zu dem der Röntgenstrahler tatsächlich in Betrieb ist und dadurch dynamische EM-Felder (z.B. wegen des Drehanodenantriebs) aussendet, die nicht auf einfache Weise kompensiert werden können.

Durch die aktive Abschirmung des Röntgenstrahlers mittels einer stromdurchflossenen Spule wird nicht nur den Einfluss des Magnetfelds des MRT-Geräts auf den Röntgenstrahler, sondern gleichzeitig auch den Einfluss des Röntgenstrahlers auf das Magnetfeld des MRT-Geräts minimiert. Es ist somit nicht mehr zwingend notwendig auf alle ferromagnetischen Materialien im Röntgenstrahler komplett zu verzichten, um die notwendige Homogenität des MRT-Magnetfelds zu gewährleisten.

Damit ist beim MRT-Gerät in vorteilhafter Weise die Beeinflussung des Magnetfelds durch den Röntgenstrahler minimiert. Außerdem können durch die aktive Abschirmung mittel- und langfristige Änderungen bezüglich auftretender Störungen weiterhin ausgeglichen werden (z.B. Ausmessen in einem Tune-Up-Schritt und Anpassen der Stromstärken in den Spulen der Abschirmeinrichtung).

Weiterhin ist bei der Röntgenröhre bzw. dem Röntgenstrahler ein kosteneffizientes Design möglich, da auf ferromagnetische Materialien nicht verzichtet werden muss.

Gemäß einer besonders bevorzugten Ausführungsform sind durch die passive Abschirmung mindestens 60 % vom MRT-Magnetfeld abschirmbar (Anspruch 8).

Bei einer weiteren vorteilhaften Ausgestaltung ist durch die aktive Abschirmung eine magnetische Flussdichte von mindestens 0,05 T abschirmbar (Anspruch 9).

Nachfolgend werden schematisch dargestellte Ausführungsbeispiele der Erfindung anhand der Zeichnung näher erläutert, ohne jedoch darauf beschränkt zu sein. Es zeigen:
- FIG 1: ein erstes Ausführungsbeispiel eines Bildgebungssystems in einem Längsschnitt,
- FIG 2: ein zweites Ausführungsbeispiel eines Bildgebungssystems in einem Längsschnitt,
- FIG 3: eine Röntgenröhre des in FIG 2 dargestellten Bildgebungssystems.

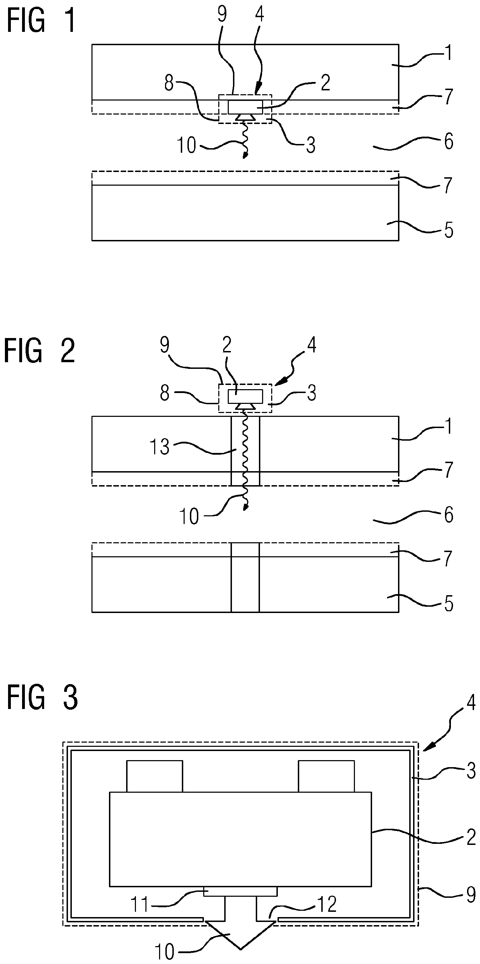

Einander entsprechende Teile sind in den FIG 1 bis 3 mit den gleichen Bezugszeichen versehen.

Die in FIG 1 und in FIG 2 dargestellten Ausführungsbeispiele eines Bildgebungssystems umfassen jeweils ein Magnetresonanztomografie(MRT)-Gerät 1 für eine MR-Bildgebung und eine Röntgenröhre 2 für eine Röntgenbildgebung.

Die Röntgenröhre 2 ist jeweils in einem Strahlergehäuse 3 angeordnet. In FIG 3 ist eine derartige Anordnung beispielhaft dargestellt. Die Röntgenröhre 2 und das Strahlergehäuse 3 bilden somit bei den gezeigten Ausführungsbeispielen einen Röntgenstrahler 4. Die Röntgenröhre 2 erzeugt im Betrieb Röntgenstrahlung 10, die zunächst durch ein Strahlenaustrittsfenster 11 aus der Röntgenröhre 2 austritt und anschließend über ein Strahlenaustrittsfenster 12 das Strahlergehäuse 3 verlässt.

Das MRT-Gerät 1 weist einen supraleitenden Magneten 5 mit einem tunnelförmigen Innenbereich 6 auf, in dem ein Patient während der Untersuchung in bekannter Weise auf einer Liege gelagert wird. Die Liege sowie der Patient sind aus Gründen der Übersichtlichkeit nicht dargestellt.

Das MRT-Gerät 1 umfasst weiterhin mehrere Hauptspulen sowie mehrere Gradientenspulen 7. Aus Übersichtlichkeitsgründen sind nur die Gradientenspulen 7 dargestellt.

Die Röntgenröhre 2 ist bei den FIG 1 und in FIG 2 gezeigten Bildgebungssystemen jeweils im Wirkungsbereich eines vom MRT-Gerät 1 erzeugten MRT-Magnetfelds angeordnet.

Durch eine Abschirmeinrichtung 8 ist sowohl die Röntgenröhre 2 während der Röntgenbildgebung vor dem MRT-Magnetfeld als auch das MRT-Gerät 1 während der MR-Bildgebung vor Einflüssen der Röntgenröhre 2 abgeschirmt.

Bei dem in FIG 1 dargestellten Bildgebungssystem ist der Röntgenstrahler 4 und damit die Röntgenröhre 2 im MRT-Gerät 1 angeordnet. Wohingegen bei dem in FIG 2 gezeigten Bildgebungssystem der Röntgenstrahler 4 bzw. die Röntgenröhre 2 außen am MRT-Gerät 1 angeordnet ist. Um einen Austritt der Röntgenstrahlung 10 aus dem Röntgenstrahler 4 in Richtung des tunnelförmigen Innenbereichs 6 zu ermöglichen, ist bei der Ausführungsform gemäß FIG 2 im supraleitenden Magneten 5 ein entsprechender Durchtrittskanal 13 angeordnet.

Die Abschirmeinrichtung 8 umfasst bei den in den FIG 1 bis 3 dargestellten Ausführungsbeispielen jeweils eine Spulengeometrie, die wenigstens eine Spule 9 umfasst und die das Strahlergehäuse 3 und damit die im Strahlergehäuse 3 angeordnete Röntgenröhre 2 in geeigneter Weise zumindest teilweise umschließt und damit das MRT-Magnetfeld (Größenordnung 2 Tesla) soweit abschirmt, dass der die Röntgenröhre 2 funktionsfähig bleibt.

Die Abschirmeinrichtung 8 schirmt damit einerseits die Röntgenröhre 2 gegenüber Einflüssen des MRT-Magnetfelds ab. Andererseits schirmt die Abschirmeinrichtung 8 das MRT-Gerät 1 während der MR-Bildgebung vor Einflüssen der Röntgenröhre 2 bzw. des Röntgenstrahlers 4 ab.

## Patentansprüche

1. Bildgebungssystem, das ein MRT-Gerät (1) für eine MR-Bildgebung und eine Röntgenröhre (2) für eine Röntgenbildgebung umfasst, wobei die Röntgenröhre (2) im Wirkungsbereich eines vom MRT-Gerät (1) erzeugten MRT-Magnetfeldes angeordnet ist und durch eine Abschirmeinrichtung (8) sowohl die Röntgenröhre (2) während der Röntgenbildgebung vor dem MRT-Magnetfeld als auch das MRT-Gerät (1) während der MR-Bildgebung vor Einflüssen der Röntgenröhre (2) zumindest teilweise abgeschirmt ist.

2. Bildgebungssystem nach Anspruch 1, wobei die Röntgenröhre (2) in dem MRT-Gerät (1) angeordnet ist.

3. Bildgebungssystem nach Anspruch 1, wobei die Röntgenröhre (2) außen an dem MRT-Gerät (1) angeordnet ist.

4. Bildgebungssystem nach Anspruch 1, wobei die Abschirmeinrichtung (8) eine Spule (9) umfasst, die die Röntgenröhre (2) zumindest teilweise umschließt.

5. Bildgebungssystem nach Anspruch 1, wobei die Röntgenröhre (2) in einem Strahlergehäuse (3) angeordnet ist.

6. Bildgebungssystem nach Anspruch 5, wobei die Abschirmeinrichtung (8) wenigstens eine Spule (9) umfasst, die das Strahlergehäuse (3) zumindest teilweise umschließt.

7. Bildgebungssystem nach Anspruch 1, wobei die Abschirmeinrichtung (8) eine passive Abschirmung und eine aktive Abschirmung umfasst.

8. Bildgebungssystem nach Anspruch 7, wobei durch die passive Abschirmung mindestens 60 % vom MRT-Magnetfeld abschirmbar sind.

9. Bildgebungssystem nach Anspruch 7, wobei durch die aktive Abschirmung eine magnetische Flussdichte von mindestens 0,05 T abschirmbar ist.
